# EUROPEAN PATENT APPLICATION

(11) **EP 4 234 599 A1**
(43) Date of publication of application: **30.08.2023**
(21) Application number: 21906895.4
(22) Date of filing: 18.11.2021
(51) Int. Cl.: C08F 290/06, C08F 220/18, C08F 2/50, A61C 13/00, A61K 6/884, B33Y 10/00, B33Y 70/00

(54) **PHOTO-CURING RESIN COMPOSITION AND MOLDED ARTICLE MANUFACTURED THEREFROM**

(30) Priority: 17.12.2020 KR 20200176999
(71) Applicant: Osstemimplant Co., Ltd., Gangseo-gu Seoul 07789 (KR)
(72) Inventor: CHOI, Giho, Seoul 07801 (KR); YANG, Bo Mi, Seoul 07609 (KR); KIM, Kyoung Rok, Seoul 07599 (KR)
(74) Representative: Beck & Rössig European Patent Attorneys
(86) International application number: PCT/KR2021/016964
(87) International publication number: WO 2022/131590

(57) **Abstract**

A photocurable resin composition including: 10 to 30 parts by weight of a (meth)acrylated urethane-based polymer; 0.1 to 30 parts by weight of a (meth)acrylated alkoxylated first bisphenol-based compound having an alkoxy/phenol molar ratio of 1 to 5; 30 to 60 parts by weight of a (meth)acrylated alkoxylated second bisphenol-based compound having an alkoxy/phenol molar ratio of 15 to 45; and 0.1 to 5 parts by weight of a photopolymerization initiator, and a molded article manufactured therefrom are disclosed.

## Description

### [Technical Field]

The present invention relates to a photocurable resin composition and a molded article manufactured therefrom.

### [Background Art]

A 3D printer is a piece of equipment that manufactures products by processing and laminating materials such as liquids, powdery resins, metal powders, and solids based on design data, and since the 3D printer can easily manufacture a molded product having a desired shape, the 3D printer is used for manufacturing prototypes or a complex-shaped molded product.

3D printer technology can be divided into a photocuring lamination method, a laser sintering lamination method, a resin extrusion lamination method, an inkjet lamination method, a polyjet lamination method, and a thin film lamination method depending on the material.

The photocuring lamination method is a method of manufacturing a molded article by curing a photocurable liquid resin with a laser beam or strong ultraviolet rays (UV), and this photocuring lamination method includes a stereo lithography apparatus (SLA), digital light processing (DLP), and light-induced planar solidification (LIPS).

The laser sintering lamination method is a method of manufacturing a three-dimensional object by sintering a powdered material with a laser beam at high pressure and high temperature, and this laser sintering lamination method includes selective laser sintering (SLS).

The resin extrusion lamination method is a method of extruding a wire-shaped material with an injection head to manufacture a three-dimensional object, and this resin extrusion lamination method includes fused deposition modeling (FDM).

The inkjet lamination method is a method of manufacturing a three-dimensional object by spraying a liquid binder from a printer head nozzle onto a material, and this inkjet lamination method includes color jetting printing (CJP).

The polyj et lamination method is a mixed form of a photocuring method and an inkjet method, in which a three-dimensional object is manufactured by spraying a material from a printer head and simultaneously curing the material with ultraviolet rays, and this polyjet lamination method includes multi jet printing (MJP)/Polyjet.

The thin film lamination method is a method of manufacturing a three-dimensional object by cutting a thin plate-shaped material with a precision cutter and then heating and bonding the material, and this thin film lamination method includes laminated object manufacturing (LOM) and paper lamination technology (PLT).

Among these various 3D printing technologies, the photocuring lamination method has excellent surface roughness characteristics and is suitable for manufacturing dental materials having complex-shaped pores such as implants. These dental materials are essential for stability under moisture conditions due to their intrinsic properties used in human teeth. However, a photopolymerization initiator used in a photocurable material has a problem in that it is decomposed or eluted when exposed to a moisture environment, causing discoloration. In particular, since a generally used phosphine oxide-based photopolymerization initiator has a unique color, it is necessary to develop a technology capable of improving such color stability.

### [Disclosure]

### [Technical Problem]

The present invention is directed to providing a photocurable resin composition with a reduced color change due to moisture and excellent color stability, and a molded article manufactured therefrom.

### [Technical Solution]

According to one aspect, the present invention provides a photocurable resin composition including: 10 to 30 parts by weight of a (meth)acrylated urethane-based polymer; 0.1 to 30 parts by weight of a (meth)acrylated alkoxylated first bisphenol-based compound having an alkoxy/phenol molar ratio of 1 to 5; 30 to 60 parts by weight of a (meth)acrylated alkoxylated second bisphenol-based compound having an alkoxy/phenol molar ratio of 15 to 45; and 0.1 to 5 parts by weight of a photopolymerization initiator.

In one embodiment, in the (meth)acrylate, at least one terminal may be modified with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate.

In one embodiment, the urethane-based polymer may be urethane dimethacrylate.

In one embodiment, the alkoxy may be one selected from the group consisting of methoxy, ethoxy, propoxy, and butoxy.

In one embodiment, the photocurable resin may further includes 1 to 10 parts by weight of a (meth)acrylate-based compound including at least one C3-C20 cyclic alkyl group.

In one embodiment, the (meth)acrylate-based compound may be isobornyl acrylate.

In one embodiment, the photopolymerization initiator may be at least one selected from the group consisting of an acetophenone-based compound, a benzophenone-based compound, a triazine-based compound, a biimidazole-based compound, a thioxanthone-based compound, an oxime ester-based compound, and a phosphine oxide-based compound.

In one embodiment, the photopolymerization initiator may be diphenyl-2,4,6-trimethylbenzoylphosphine oxide or phenylbis(2,4,6-trimethylbenzoyl)-phosphine oxide.

In one embodiment, the photocurable resin composition may be a liquid composition used for 3D printing of a digital light processing method, a stereo lithography apparatus method, or a light-induced planar solidification method.

In one embodiment, the composition may be photocured at a wavelength of 360 to 405 nm.

According to another aspect, a molded article manufactured by irradiating the above-described photocurable resin composition with light is provided.

### [Advantageous Effects]

According to one aspect, the present invention can provide a photocurable resin composition having excellent color stability by reducing a color change due to moisture, and a molded article manufactured therefrom.

The effect of one aspect of the present specification is not limited to the above effect, and it should be understood to include all effects that can be inferred from the configuration described in the detailed description or claims of the present specification.

### [Modes of the Invention]

Hereinafter, one aspect of the present specification will be described with reference to each specific embodiment. However, the description of the present specification may be implemented in several different forms, and thus is not limited to the embodiments described herein.

Throughout the specification, when a part is "connected" to another part, this includes not only the case where it is "directly connected" but also the case where it is "indirectly connected" with another member interposed therebetween. In addition, when a part is said to "include" a component, this means that other components may be further included, not excluded, unless specifically stated otherwise.

When a range of numerical values is recited herein, the values have the precision of the significant figures provided in accordance with the standard rules in chemistry for significant figures, unless the specific range is otherwise stated. For example, 10 includes the range of 5.0 to 14.9, and the number 10.0 includes the range of 9.50 to 10.49.

In the present specification, "(meth)acryl-" means "methacryl-," "acryl-" or both.

### Photocurable resin composition

The photocurable resin composition according to one aspect may include: 10 to 30 parts by weight of a (meth)acrylated urethane-based polymer; 0.1 to 30 parts by weight of a (meth)acrylated alkoxylated first bisphenol-based compound having an alkoxy/phenol molar ratio of 1 to 5; 30 to 60 parts by weight of a (meth)acrylated alkoxylated second bisphenol-based compound having an alkoxy/phenol molar ratio of 15 to 45; and 0.1 to 5 parts by weight of a photopolymerization initiator.

When the photocurable resin composition is exposed to moisture after curing, the photopolymerization initiator is eluted or decomposed to cause a color change, so that it is possible to improve a problem of deterioration in color stability.

In the (meth)acrylate, at least one terminal may be modified with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate. Examples of such (meth)acrylates may be represented by the formula below.

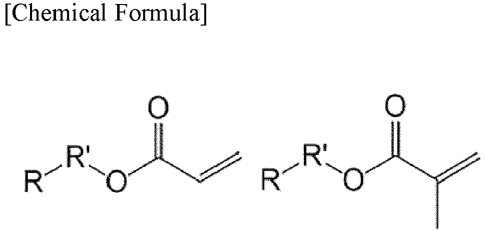

In the above formula, R is a polymer or compound to be (meth)acrylated, and R' may be a single bond or methylene, ethylene, propylene, or butylene. In the above chemical formula, the compound on the left means acrylation, and the compound on the right means methacrylation.

The content of the urethane-based polymer may be 10 to 30 parts by weight, for example, 10 parts by weight, 15 parts by weight, 20 parts by weight, 25 parts by weight, 30 parts by weight, or a range between two of these values, and when the content of the urethane-based polymer is outside the above range, the mechanical properties of the molded article may deteriorate. The (meth)acrylated urethane-based polymer may be urethane dimethacrylate, but is not limited thereto. The composition can improve color stability by simultaneously including a first bisphenol-based compound, which is a low alkoxylated bisphenol-based compound having a low alkoxy/phenol molar ratio, and a second bisphenol-based compound, which is a high alkoxylated bisphenol-based compound having a high alkoxy/phenol molar ratio. The content of the first bisphenol-based compound may be 0.1 to 30 parts by weight, for example, 0.1 parts by weight, 1 part by weight, 5 parts by weight, 10 parts by weight, 15 parts by weight, 20 parts by weight, 25 parts by weight, 30 parts by weight, or a range between two of these values, and the content of the second bisphenol-based compound may be 30 to 60 parts by weight, for example, 30 parts by weight, 35 parts by weight, 40 parts by weight, 45 parts by weight, 50 parts by weight, 55 parts by weight, 60 parts by weight, or a range between two of these values. When the contents of the first and second bisphenol-based compounds are outside the above ranges, color stability may deteriorate or photocuring by a light source may be disadvantageous, which may be disadvantageous to 3D printing.

The alkoxy/phenol molar ratio may represent, for example, an EO/phenol value, which is a molar ratio of ethylene oxide to phenol, but is not limited thereto.

The alkoxy/phenol molar ratio is 1 to 5, the (meth)acrylated alkoxylated first bisphenol-based compound is ethoxylated bisphenol A dimethacrylate having an ethylene oxide mole number of 2, and for example, a commercially available ethoxylated bisphenol A dimethacrylate (EO/phenol 2) may be used, but is not limited thereto.

The alkoxy/phenol molar ratio is 15 to 45, the (meth)acrylated alkoxylated second bisphenol-based compound is ethoxylated bisphenol A dimethacrylate having an ethylene oxide mole number of 30, and for example, a commercially available ethoxylated bisphenol A dimethacrylate (EO/phenol 30) may be used, but is not limited thereto.

A molar ratio between the first bisphenol-based compound and the second bisphenol-based compound may be 1:0.5 to 1.5. When the molar ratio of the bisphenol-based compounds is outside the above range, color stability may deteriorate.

The alkoxy may be one selected from the group consisting of methoxy, ethoxy, propoxy, and butoxy. The bisphenol-based compound may be bisphenol A, bisphenol F, or bisphenol S, but is not limited thereto.

The composition may further include a (meth)acrylate-based compound. The (meth)acrylate-based compound includes at least one C3-C20 cyclic alkyl group, and when the cyclic alkyl group is included, the curing speed and curing power for a photopolymerization initiator may be excellent. In addition, decomposition or elution of the photopolymerization initiator when exposed to moisture can be prevented.

The content of the (meth)acrylate-based compound is 1 to 10 parts by weight, for example, 1 part by weight, 2 parts by weight, 3 parts by weight, 4 parts by weight, 5 parts by weight, 6 parts by weight, 7 parts by weight, 8 parts by weight, 9 parts by weight, 10 parts by weight, or a range between two of these values.

In one example, the (meth)acrylate-based compound may be isobornyl acrylate, but is not limited thereto.

The photopolymerization initiator may be at least one selected from the group consisting of an acetophenone-based compound, a benzophenone-based compound, a triazine-based compound, a biimidazole-based compound, a thioxanthone-based compound, an oxime ester-based compound, and a phosphine oxide-based compound, for example, may be diphenyl-2,4,6-trimethylbenzoylphosphine oxide or phenylbis(2,4,6-trimethylbenzoyl)-phosphine oxide, but is not limited thereto.

The photopolymerization initiator may be eluted or decomposed in a moisture environment to cause a color change in a cured product, but the composition may prevent such a color change by simultaneously including a urethane-based polymer, a first bisphenol-based compound with a low alkoxy/phenol molar ratio, and a second bisphenol-based compound with a high alkoxy/phenol molar ratio.

The photocurable resin composition may be a liquid composition used for 3D printing of a digital light processing (DLP) method, a stereo lithography apparatus (SLA) method, or a light-induced planar solidification (LIPS) method. Accordingly, the 3D printing may be performed using a printer using a liquid resin. A photocurable 3D printer is a printer that cures a material by irradiating light to an area where an output is desired, and has excellent surface roughness compared to other printing methods and is advantageous for manufacturing complex structures.

In the stereo lithography apparatus method, a molded article may be prepared by irradiating an ultraviolet laser onto a water tank containing a photocurable resin composition, curing the composition, and laminating the composition. In such SLA 3D printing, the wavelength of irradiated laser may be changed depending on the type of composition, and the curing speed, the strength of the cured molded article, and surface roughness may vary.

The digital light processing method is a mask projection image curing method, and a molded article having a desired shape can be manufactured by selectively projecting light onto a photocurable resin and curing the resin. Generally, unlike manufacturing a product with a descending molding plate, the molding plate moves upward and products can be produced in a downward direction. At this time, the molded article may be prepared by projecting the light provided from a beam projector onto the curing resin composition for 3D printing. That is, curing is sequentially performed on the molding plate in units of slice cross-section layers, and a 3D molded article can be prepared.

The light-induced planar solidification method is a method of selectively projecting light onto a photocurable resin using a flat light source such as an LCD or LED to cure the resin, and can perform uniform photocuring regardless of an area by preventing the wavelength bending phenomenon of light according to the curvature of the lens, unlike the digital light processing method.

The photocurable resin composition may be photocured at a wavelength of 360 to 405 nm.

The photocurable resin composition can be used for 3D printing and used for manufacturing various dental materials. In particular, according to one embodiment, by using a photocurable resin composition that satisfies a specific composition, it is possible to implement excellent color stability while satisfying the strength required for dental materials. If a composition of the composition is outside the above range, a sufficient cross-linking structure may not be formed by photocuring alone, and thus required physical properties may not be satisfied, or a color change may occur under moisture conditions, resulting in insufficient aesthetics.

### Molded article

A molded article according to another aspect can be prepared by irradiating the photocurable resin composition described above with light.

The light irradiation may be performed by using a known SLA, DLP, or LIPS method.

The molded article may be prepared by 3D printing using a digital light processing method, a stereo lithography apparatus method, or a light-induced planar solidification method. Therefore, the molded article may have a smoother surface and a more complex structure than a molded article manufactured by dissolving a filament using a fusion modeling method.

The molded article may be a dental material. When the molded article is prepared by irradiating the photocurable resin composition with light, it is possible to sufficiently realize the mechanical strength required for dental materials, and at the same time, the stability against moisture is improved, so that it is possible to solve the problem of conventional dental materials that have poor aesthetics due to color changes under practical conditions.

Hereinafter, examples of the present specification will be described in more detail. However, the following experimental results describe only representative experimental results among the examples, and the scope and content of the present specification may not be construed as reduced or limited by the examples. Each effect of the various embodiments of the present specification not explicitly presented below will be specifically described in the corresponding section.

### Examples 1 to 7 and Comparative Examples 1 to 5

Urethane dimethacrylate, ethoxylated bisphenol A dimethacrylate having an ethylene oxide mole number of 2, ethoxylated bisphenol A dimethacrylate having an ethylene oxide mole number of 30, isobornyl acrylate, and phenylbis (2,4,6-trimethylbenzoyl)-phosphine oxide were mixed.

**[Table 1]**

| Classification | Urethane | Bisphenol A (EO=2) | Bisphenol A (EO=30) | Isobornyl acrylate | Photoinitiator |
|---|---|---|---|---|---|
| Example 1 | 20 | 9 | 60 | 0 | 1 |
| Example 2 | 20 | 19 | 50 | 0 | 1 |
| Example 3 | 20 | 29 | 40 | 0 | 1 |
| Example 4 | 20 | 29 | 40 | 10 | 1 |
| Example 5 | 30 | 19 | 30 | 10 | 1 |
| Example 6 | 30 | 19 | 30 | 10 | 0.5 |
| Comparative Example 1 | 20 | 60 | 0 | 0 | 1 |
| Comparative Example 2 | 20 | 50 | 60 | 0 | 1 |
| Comparative Example 3 | 20 | 40 | 30 | 0 | 1 |
| Comparative Example 4 | 30 | 60 | 0 | 10 | 1 |
| Comparative Example 5 | 30 | 50 | 30 | 10 | 1 |
| Comparative Example 6 | 30 | 40 | 30 | 10 | 1 |
| Comparative Example 7 | 30 | 40 | 30 | 0 | 10 |
| Comparative Example 8 | 30 | 30 | 30 | 5 | 6.5 |

### Comparative Example 9

10 parts by weight of urethane dimethacrylate, 50 parts by weight of ethoxylated bisphenol A dimethacrylate having an ethylene oxide mole number of 4, 10 parts by weight of triethylene glycol dimethacrylate, 10 parts by weight of tetrahydrofurfuryl methacrylate, 1 part by weight of phenylbis (2,4,6-trimethylbenzoyl)-phosphine oxide, and 5 parts by weight of camphorquinone were mixed.

### Experimental Example 1

Disk specimens having a diameter of 10 mm and a thickness of 2 mm were printed using a 3D printing device equipped with a light source of 360 to 405 nm for the compositions of Examples and Comparative Examples. After washing the specimen with an isopropyl alcohol washing solution, it was cured for 10 to 30 minutes with a post-curing machine.

The specimen was exposed to various environments for 2 weeks, and color stability was measured by analyzing a color difference (ΔE) before and after exposure of the specimen using a spectrophotometer.
- First condition: 25°C
- Second condition: relative humidity of 80% at 40°C
- Third condition: in a 40 °C PBS solution

**[Table 2]**

| Classification | Condition 1 | Condition 2 | Condition 3 |
|---|---|---|---|
| Example 1 | ○ | ○ | ○ |
| Example 2 | ○ | ○ | ○ |
| Example 3 | ○ | ○ | ○ |
| Example 4 | ○ | ○ | ○ |
| Example 5 | ○ | ○ | ○ |
| Example 6 | ○ | ○ | ○ |
| Example 7 | ○ | ○ | △ |
| Comparative Example 1 | × | × | × |
| Comparative Example 2 | × | × | × |
| Comparative Example 3 | △ | △ | × |
| Comparative Example 4 | × | × | × |
| Comparative Example 5 | ○ | △ | △ |
| Comparative Example 6 | ○ | △ | △ |
| Comparative Example 7 | × | × | × |
| Comparative Example 8 | △ | × | × |
| Comparative Example 9 | ○ | △ | × |

| | | | |
|---|---|---|---|
| (∘: good, △: insufficient, and ×: poor) | | | |

Referring to Table 2, Examples 1 to 6 combining urethane with low ethoxylated bisphenol A and high ethoxylated bisphenol A showed excellent color stability under high humidity conditions or in a buffer solution similar to the body, but Comparative Examples 1 to 9 had insufficient color stability under a moisture environment.

In addition, Examples 4 to 6 further including isobornyl acrylate showed excellent mechanical strength and color stability of a cured product while reducing the curing time to less than half compared to Examples 1 to 3.

The description of the present specification described above is for illustration, and it should be understood that those of ordinary skill in the art to which one aspect of the present specification belongs can easily modify it into other specific forms without changing the technical idea or essential features described in this specification. Therefore, it should be understood that the embodiments described above are illustrative in all respects and not restrictive. For example, each component described as a single type may be implemented in a distributed form, and likewise, components described as distributed may be implemented in a combined form.

The scope of the present specification is indicated by the following claims, and all changes or modifications derived from the meaning and scope of the claims and their equivalents should be construed as being included in the scope of the present specification.

## Claims

1. A photocurable resin composition comprising:
10 to 30 parts by weight of a (meth)acrylated urethane-based polymer;
0.1 to 30 parts by weight of a (meth)acrylated alkoxylated first bisphenol-based compound having an alkoxy/phenol molar ratio of 1 to 5;
30 to 60 parts by weight of a (meth)acrylated alkoxylated second bisphenol-based compound having an alkoxy/phenol molar ratio of 15 to 45; and
0.1 to 5 parts by weight of a photopolymerization initiator.

2. The photocurable resin composition of claim 1, wherein in the (meth)acrylate, at least one terminal is modified with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate.

3. The photocurable resin composition of claim 1, wherein the urethane-based polymer is urethane dimethacrylate.

4. The photocurable resin composition of claim 1, wherein the alkoxy is one selected from the group consisting of methoxy, ethoxy, propoxy, and butoxy.

5. The photocurable resin composition of claim 1, further comprising 1 to 10 parts by weight of a (meth)acrylate-based compound including at least one C3-C20 cyclic alkyl group.

6. The photocurable resin composition of claim 5, wherein the (meth)acrylate-based compound is isobornyl acrylate.

7. The photocurable resin composition of claim 1, wherein the photopolymerization initiator is at least one selected from the group consisting of an acetophenone-based compound, a benzophenone-based compound, a triazine-based compound, a biimidazole-based compound, a thioxanthone-based compound, an oxime ester-based compound, and a phosphine oxide-based compound.

8. The photocurable resin composition of claim 7, wherein the photopolymerization initiator is diphenyl-2,4,6-trimethylbenzoylphosphine oxide or phenylbis(2,4,6-trimethylbenzoyl)-phosphine oxide.

9. The photocurable resin composition of claim 1, which is a liquid composition used for 3D printing of a digital light processing method, a stereo lithography apparatus method, or a light-induced planar solidification method.

10. The photocurable resin composition of claim 1, which is photocured at a wavelength of 360 to 405 nm.

11. A molded article manufactured by irradiating the photocurable resin composition according to any one of claims 1 to 10 with light.
